# FASCICULE DE BREVET EUROPEEN

(11) **EP 0 688 197 B1**
(45) Date de publication et mention de la délivrance du brevet: **08.09.1999**
(21) Numéro de dépôt: 95903743.3
(22) Date de dépôt: 30.12.1994
(51) Int. Cl.: A61F 2/06

(54) **PROTHESE ELASTIQUE POUR ELARGIR UN CONDUIT, NOTAMMENT UN VAISSEAU SANGUIN, ET SON PROCEDE DE FABRICATION**
ELASTISCHE PROTHESE ZUR VERBREITERUNG EINES GEFÄSSES, INSBESONDERE EINES BLUTGEFÄSSES, UND VERFAHREN ZU DEREN HERSTELLUNG
RESILIENT PROSTHESIS FOR WIDENING A CHANNEL, PARTICULARLY A BLOOD VESSEL, AND METHOD FOR MAKING SAME

(30) Priorité: 10.01.1994 FR 9400302
(43) Date de publication de la demande: 27.12.1995
(73) Titulaire: MICROFIL INDUSTRIES S.A., 1020 Renens (CH)
(72) Inventeur: BLANC, Louis, CH-1095 Lutry (CH)
(74) Mandataire: Nithardt, Roland
(86) Numéro de dépôt international: CH9400247
(87) Numéro de publication internationale: WO9518585

(56) Documents cités:
- WO-A-83/00997
- DE-B- 1 766 921
- US-A- 4 300 244

## Description

La présente invention concerne une prothèse élastique pour élargir un conduit d'un être vivant, notamment un vaisseau sanguin, la prothèse ayant une forme générale allongée sensiblement cylindrique.

L'invention concerne également un procédé de fabrication d'une telle prothèse.

Ce genre de prothèse est souvent désigné par le terme américain "stent" et est utilisable en particulier, mais pas exclusivement, dans les vaisseaux sanguins présentant des sténoses, notamment les artères coronaires. Comme le diamètre intérieur naturel du vaisseau est réduit dans la zone sténosée, on effectue de plus en plus couramment un traitement d' angioplastie consistant à élargir ce diamètre au moyen d'un ballon gonflable disposé sur un cathéter, puis à remplacer ce ballon par une prothèse élastique permanente dont le diamètre au repos est supérieur à celui du vaisseau, de sorte que la prothèse reste pressée en permanence contre la paroi intérieure du vaisseau. Pour mettre la prothèse en place, on la comprime pour réduire son diamètre et l'insérer dans l'extrémité d'un tube que l'on amène à l'endroit voulu, puis on la chasse hors du tube pour qu'elle se dilate par son élasticité propre.

Les publications EP-A-0 183 372 et WO 83/03752 décrivent des prothèses de ce genre, composées d'un treillis de filaments élastiques tressés ou tissés ensemble. De plus, les déformations et mouvements de la prothèse en cours d' utilisation créent des frictions entre les fils aux points de croisement et ceci empêche d'utiliser des filaments métalliques pourvus de revêtements spéciaux destinés à prévenir des dépôts. Ces prothèses sont relativement coûteuses. Une prothèse plus simple, décrite par exemple dans le WO 83/00997, est constituée d'un seul fil enroulé sous une configuration analogue à un ressort hélicoïdal. Pour mettre cette prothèse en place, il est possible de réduire son diamètre en la tordant et de la fixer ainsi sur un instrument qui sert à l'amener dans le conduit ou vaisseau à élargir. Toutefois, il s'est avéré que les prothèses de ce type sont susceptibles de se déplacer longitudinalement par reptation à cause des contraintes que le vaisseau doit exercer sur elles, spécialement dans la zone où le diamètre naturel du vaisseau est réduit. Ce phénomène se produit par glissement longitudinal du fil le long de sa trajectoire hélicoïdale. La principale résistance à ce mouvement, à part la friction qui est relativement minime sur les parois du vaisseau, vient de l'extrémité du fil qui tend à s'accrocher à la paroi du vaisseau; cette résistance est assez faible, en regard de l'effort d'élargissement exercé sur le vaisseau dans la zone sténosée.

La présente invention vise à éviter les inconvénients mentionnés ci-dessus, en créant une prothèse susceptible d'être réalisée d'une manière peu coûteuse, d'exercer un effort relativement fort dans la zone du conduit ou vaisseau qui doit être élargie et de présenter un faible risque de reptation après sa mise en place.

Dans ce but, un premier aspect de l'invention concerne une prothèse du genre indiqué plus haut étant composée de plusieurs segments de fil élastique ayant une configuration en spires hélicoïdales, caractérisée en ce que le sens des spires est le même pour tous les segments de fil, et en ce qu'en dehors des zones d'extrémité de la prothèse, les segments de fil sont intercalés les uns entre les autres sans se croiser, de manière à former ensemble ladite forme générale sensiblement cylindrique.

De cette manière, il est possible de choisir le nombre de segments de fil de la prothèse pour lui donner les caractéristiques mécaniques voulues et en particulier les forces d' expansion suffisantes, sans que sa construction soit coûteuse puisque tous les segments de fil peuvent être semblables et n'ont pas besoin d'être fixés les uns aux autres. S'il le faut, chaque segment de fil peut avoir une extrémité libre à chaque extrémité de la prothèse, de sorte que l'accrochage longitudinal de la prothèse dans le conduit est amélioré. Dans d'autres cas, les segments de fil peuvent être liés entre eux a chaque extrémité de la prothèse, mais ils peuvent former tout de même plusieurs points d' accrochage à chaque extrémité.

Dans une forme de réalisation particulièrement avantageuse, les spires de chaque segment de fil ont un pas d' hélice plus court dans une partie médiane que dans le reste de la longueur de la prothèse. De ce fait, la prothèse est plus rigide dans sa partie médiane, qui correspond de préférence à la zone à élargir dans le vaisseau ou conduit, c'est-à-dire qu'elle peut exercer une pression plus forte sur cette zone. Par contre elle est plus souple dans ses parties terminales, situées dans des zones saines du vaisseau ou conduit et servant avant tout à maintenir la prothèse en place. De préférence, le pas d' hélice dans le reste de la longueur vaut au moins 1,5 fois le pas d' hélice dans la partie médiane.

Dans une première forme de réalisation, tous les segments de fil sont identiques. Ils peuvent avoir chacun deux extrémités libres et ne pas être liés les uns aux autres.

A une extrémité de la prothèse, les extrémités libres des segments de fil peuvent se trouver sensiblement dans un même plan transversal. Dans une autre forme de réalisation, à une extrémité de la prothèse, les extrémités libres des segments de fil sont décalées axialement les unes par rapport aux autres.

De préférence, le nombre de segments de fil composant la prothèse est compris entre 4 et 18.

Dans une forme de réalisation particulière, les segments de fil peuvent être liés mutuellement au moins deux à deux aux extrémités de la prothèse. Lesdits segments de fil peuvent être liés par soudage. Autrement, au moins certains des segments de fil peuvent être formés d'un seul fil continu qui est replié ou recourbé à 180° à chaque extrémité de la prothèse pour raccorder à chaque fois deux desdits segments.

Un autre aspect de la présente invention concerne un procédé pour fabriquer une telle prothèse où certains ou, de préférence, tous les segments de fil sont formés d'un seul fil continu.

Une première forme du procédé comporte les étapes suivantes :
- on utilise des moyens de support comprenant un premier et un second support à pourtour circulaire, qui sont alignés axialement l'un en face de l'autre et ont chacun une série d'éléments saillants répartis sur le pourtour du support, et un mandrin central disposé axialement entre les deux séries d'éléments saillants;
- on dépose le fil sur les moyens de support suivant un tracé en zig-zag par un mouvement en va-et-vient axial entre les deux séries d'éléments saillants pour former un ensemble de segments de fil axiaux, parallèles et répartis autour du mandrin, le fil étant replié successivement sur au moins un élément saillant du premier support, puis du second support, puis sur au moins un autre élément saillant du premier support, et ainsi de suite;
- on coupe le fil pour former deux extrémités adjacentes et on fixe lesdites extrémités l'une à l'autre;
- on tord ledit ensemble de segments de fil autour du mandrin pour donner à chaque segment de fil une configuration permanente en spires hélicoïdales et l'on enlève des moyens de support ledit ensemble qui forme la prothèse.

Une seconde forme du procédé comporte les étapes suivantes :
- on utilise des moyens de support comprenant un premier et un second support à pourtour circulaire, qui sont alignés axialement l'un en face de l'autre et ont chacun une série d'éléments saillants répartis sur le pourtour du support, et un mandrin central disposé axialement entre les deux séries d'éléments saillants;
- on dépose le fil sur les moyens de support suivant un tracé en zig-zag par un mouvement en va-et-vient hélicoïdal entre les deux séries d'éléments saillants, ou par un mouvement en va-et-vient axial combiné avec un mouvement alternatif de rotation des moyens de support, pour former un ensemble de segments de fil parallèles et enroulés en spires hélicoïdales autour du mandrin, le fil étant replié successivement sur au moins un élément saillant du premier support, puis du second support, puis sur au moins un autre élément saillant du premier support, et ainsi de suite;
- on coupe le fil pour former deux extrémités adjacentes et on fixe lesdites extrémités l'une à l'autre; et
- on enlève des moyens de support ledit ensemble qui forme la prothèse.

D'autres caractéristiques et avantages de la présente invention apparaîtront dans la description suivante de divers exemples de réalisation, en référence aux dessins annexés, dans lesquels :
la figure 1 est une vue schématique en élévation latérale d'une première forme de réalisation d'une prothèse d'élargissement selon l'invention,
la figure 2 est une vue schématique en coupe longitudinale illustrant un procédé de mise en place de la prothèse de la figure 1,
la figure 3 est une vue latérale d'une extrémité d'une prothèse d' élargissement, dans une autre forme de réalisation de l'invention,
la figure 4 est une vue analogue à la figure 2, représentant une autre forme du dispositif de mise en place,
la figure 5 représente à une échelle agrandie un détail de la figure 4,
la figure 6 est une vue analogue à la figure 5 et représente une autre position du dispositif,
les figures 7 et 8 sont des vues latérales illustrant deux étapes successives du procédé de fabrication d'une autre forme de prothèse selon l'invention,
la figure 9 est une vue latérale agrandie de la prothèse ainsi fabriquée, et
la figure 10 est une vue analogue à la figure 7, illustrant la fabrication d'une autre forme de prothèse selon l'invention.

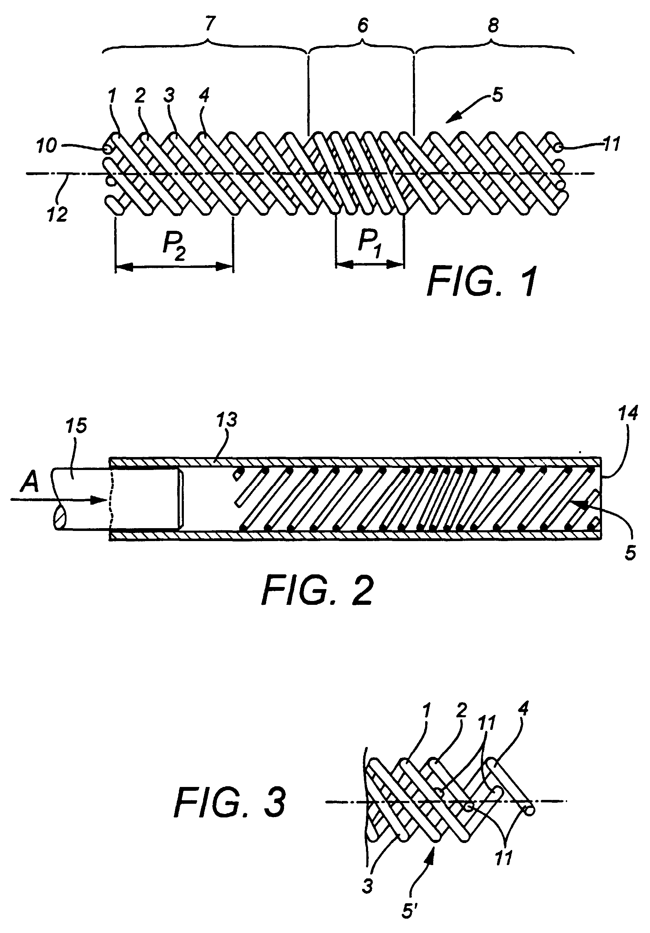

En référence à la figure 1, quatre fils métalliques élastiques 1, 2, 3, 4, c'est-à-dire quatre segments de fil, forment ensemble une prothèse élastique 5 (ou "stent") pour élargir un vaisseau sanguin ou un autre conduit d'un être vivant. La prothèse est représentée dans son état libre ou de repos, où elle a une forme générale cylindrique dont le diamètre est légèrement supérieur au diamètre intérieur naturel du vaisseau concerné. Les quatre fils 1 à 4 sont identiques les uns aux autres; chacun d'eux est enroulé en spires hélicoïdales, mais le pas des spires n'est pas le même sur toute la longueur de la prothèse 5. Dans une partie médiane 6 destinée à être placée dans la zone à élargir du vaisseau, les spires ont un pas P₁ relativement court, légèrement supérieur à quatre fois la largeur du fil, de sorte qu'elles sont presque jointives dans cette zone. Par contre, dans deux parties adjacentes 7 et 8 qui forment les zones d'extrémité de la prothèse 5, les spires présentent un pas P₂ plus grand que P₁, de sorte qu'elles sont plus espacées axialement les unes des autres. Il en résulte que la prothèse 5 présente une plus grande rigidité diamétrale dans la partie médiane 6 que dans les autres parties 7 et 8, c'est-à-dire que dans le vaisseau où son diamètre est réduit par compression, elle exercera un plus grand effort diamétral dans la zone de cette partie 6 où il s'agit d'élargir spécialement le vaisseau. Selon les cas, le pas des spires peut varier brusquement ou progressivement de la partie 6 à chaque partie adjacente 7 et 8, et il peut éventuellement être différent dans les deux parties 7 et 8.

Les fils 1, 2, 3, 4 ont chacun deux extrémités libres 10 et 11 qui peuvent s'accrocher à la paroi intérieure du vaisseau pour empêcher un déplacement longitudinal du fil, donc aussi de l'ensemble de la prothèse 5. Dans cet exemple, les quatre extrémités 10 se trouvent dans un même plan transversal, et il en va de même pour les quatre extrémités 11. Ces extrémités peuvent être arrondies ou coupées à angle vif selon les cas. Bien entendu, l'effet de retenue obtenu sur quatre extrémités 10, 11 des fils à chaque extrémité de la prothèse est quatre fois plus fort qu'avec les prothèses connues faites d'un seul fil.

Les fils 1, 2, 3, 4 ne sont pas fixés les uns aux autres. Cependant, ils sont retenus les uns dans les autres grâce aux différences de pas d' hélice entre les parties 6, 7 et 8 de la prothèse car cette différence empêche qu'un fil puisse se déplacer longitudinalement dans une mesure substantielle en se "vissant" entre les autres. En fait, chaque fil dispose d'un faible jeu entre les autres dans la partie médiane 6, ce qui lui permet éventuellement de tourner d'une fraction de tour autour de l'axe longitudinal 12 de la prothèse, après quoi il est arrêté par contact avec les fils adjacents dans les autres parties 7 et 8. Dans ce but, il est préférable que P₂ soit égal ou supérieur à 1,5 fois P₁. Le même phénomène intervient si un fil, mal retenu par son extrémité 10 ou 11, tend à cheminer longitudinalement dans le vaisseau, c'est-à-dire que la prothèse 5 offre d'excellentes garanties de rester à sa place pendant une longue durée d' utilisation. Par contre, il est possible de la retirer chirurgicalement sans difficulté. Il suffit de retirer chaque fil l'un après l'autre en le saisissant au moyen d'une pince à une extrémité et en le tirant en tournant, ce qui réduit le diamètre de ses spires et permet de le faire passer à l'intérieur des autres spires pour l'extraire.

Du fait que tous les fils 1 à 4 sont identiques on peut fabriquer la prothèse 5 d'une manière très simple, en enroulant ensemble les quatre fils parallèlement sur un mandrin de diamètre approprié pour les façonner. Les quatre fils peuvent être très proches les uns des autres pendant l'opération d'enroulement et dans la prothèse finie, et chaque groupe de quatre spires ainsi formées peut être séparé des groupes voisins par un intervalle qui varie lorsque le pas des spires varie le long de la prothèse. On peut aussi envisager de façonner les fils séparément et de les intercaler ensuite. L'absence de fixation entre les fils facilite considérablement la fabrication. De plus s'il le juge opportun, le chirurgien peut aussi enlever un fil avant de mettre la prothèse en place.

La figure 2 montre schématiquement un dispositif pour la mise en place de la prothèse d' élargissement 5 dans un vaisseau. La prothèse 5 est introduite dans un cathéter 13 ayant une extrémité à bord arrondi 14 et un diamètre intérieur plus petit que le diamètre extérieur de la prothèse au repos, de sorte que la prothèse 5 est comprimée diamétralement dans le cathéter. Comme tous les fils sont enroulés dans le même sens, il est facile de tordre la prothèse pour réduire élastiquement son diamètre afin de la placer dans le cathéter 13. La prothèse 5 ayant toujours une forme tubulaire, il est possible de la traverser au moyen d'un autre appareil, par exemple un fil de guidage et/ou un cathéter à ballon d'angioplastie. Une fois que l'extrémité 14 du cathéter a été amenée dans la partie du vaisseau à élargir, on en chasse la prothèse 5 au moyen d'un mandrin 15 à tête cylindrique, que l'on pousse en avant suivant la flèche A pendant que l'on retire progressivement le cathéter 13.

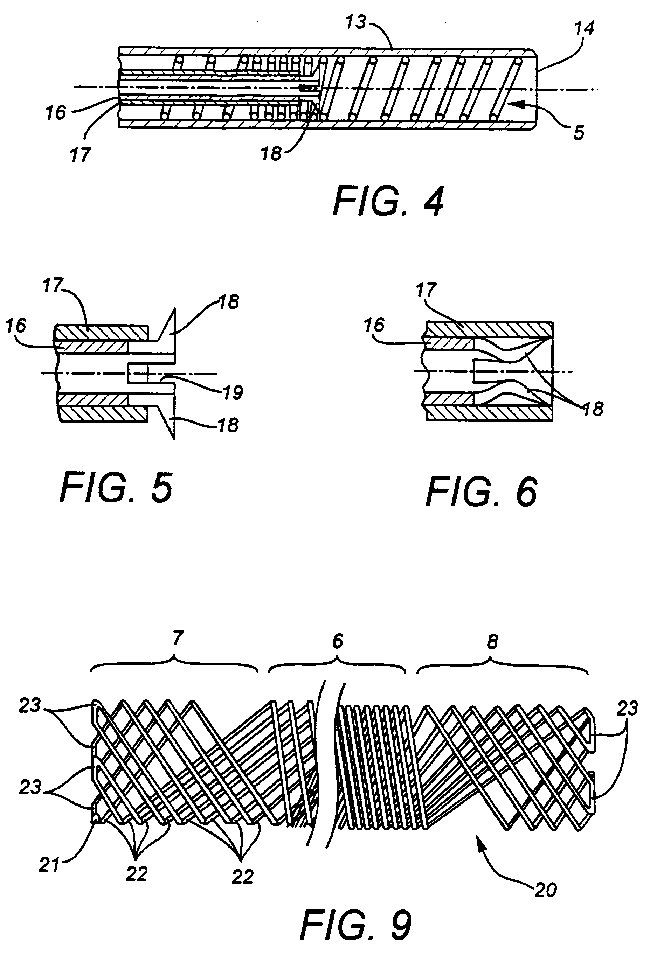

Il s'est avéré que le dispositif représenté à la figure 2 ne fonctionne bien qu'à condition que les spires soient assez fortement inclinées par rapport à un plan radial, sinon elles ont tendance à se coincer dans le cathéter 13. Le dispositif représenté aux figures 4 à 6 permet d'éviter cet inconvénient. Dans ce cas, le mandrin 15 de la figure 2 est remplacé par un tube de poussée 16 disposé de manière coulissante dans un tube extérieur 17. L'extrémité avant du tube de poussée 16 est façonnée pour présenter par exemple quatre ergots coudés 18 qui, dans la position illustrée par les figures 4 et 5, sont proéminents latéralement par rapport au tube extérieur 17 et peuvent ainsi s'engager entre deux spires successives de la prothèse 5 logée dans le cathéter 13. Les ergots 18 sont flexibles et séparés par des encoches axiales 19. Dans la position initiale illustrée par la figure 5, le tube extérieur 17 les empêche de fléchir vers l'extérieur. Avec ce dispositif une fois que l'extrémité avant du cathéter 13 a franchi le site où la prothèse 5 doit être placée dans un conduit on commence à retirer le cathéter 13 tout en maintenant la position des tubes 16 et 17 par rapport au site de sorte que la prothèse 5 est tirée hors du cathéter 13 par les ergots 18 et prend place dans le conduit au fur et à mesure de sa libération à l'extrémité du cathéter 13. Une fois que la prothèse 5 est entièrement sortie du cathéter 13, on escamote les ergots 18 comme le montre la figure 6 par un mouvement relatif entre les tubes 16 et 17, puis on retire ces deux tubes à l'intérieur du cathéter 13.

D'autres modes de mise en place d'une prothèse selon l'invention peuvent être envisagés, par exemple avec le dispositif d'application décrit dans la publication WO 83/00997, en l'utilisant pour mettre en place non pas un seul fil hélicoïdal, mais plusieurs fils ou segments de fil hélicoïdaux formant ensemble une prothèse selon l'invention.

La prothèse 5 décrite ci-dessus peut comporter n'importe quel nombre de fils à partir de deux. Avec deux fils seulement les pas différents P₁ et P₂ permettent aussi d'obtenir une retenue longitudinale d'un fil par rapport à l'autre, mais le jeu angulaire est relativement important. On peut envisager jusqu'à environ une vingtaine de fils intercalés pour former la prothèse, mais un nombre de fils trop élevé est peu avantageux parce qu'il implique un pas relativement grand et donc une prothèse relativement longue pour que chaque fil comporte plusieurs spires. Par contre, plus les fils sont nombreux meilleure est la retenue par leurs extrémités. A l'heure actuelle, on estime que le nombre optimal de fils est de l'ordre de quatre à dix-huit ou même vingt-quatre, selon les cas particuliers à traiter. Des essais ont démontré la possibilité de fabriquer une telle prothèse par enroulements successifs de plusieurs groupes de fils métalliques juxtaposés.

La figure 3 montre une variante de réalisation d'une prothèse 5' généralement semblable à la prothèse 5, mais où les fils 1, 2, 3, 4 n'ont pas tous la même longueur de sorte que leurs extrémités respectives 11 sont décalées les unes par rapport aux autres dans la direction axiale de la prothèse. Ainsi, elles ne se trouvent pas toutes dans la même partie du vaisseau si bien qu'elles peuvent mieux s'accrocher contre la paroi du vaisseau et exercer un traumatisme moindre que si elles agissaient toutes au même endroit.

Un autre aspect avantageux des prothèses 5 et 5' décrites ci-dessus est à voir dans leur grande flexibilité axiale et diamétrale dans les zones d'extrémité 7 et 8. Cela permet d'appuyer moins fort sur des zones saines de la paroi du vaisseau, donc de réduire un risque de réaction des tissus du vaisseau. En outre, il est possible d'avoir une prothèse plus longue pour que les points d'appui (aux extrémités) soient plus éloignés de la zone sténosée qui est la plus sollicitée. Ces zones particulièrement flexibles 7 et 8 peuvent même se trouver dans un coude d'un vaisseau, ce qui n'est en général pas possible avec les prothèses habituelles.

Dans les cas où l'on craint que les extrémités libres 10 et 11 des fils puissent traumatiser la paroi du vaisseau ou conduit concerné, on peut prévoir de souder ces extrémités deux à deux, ou trois à trois etc., pour former des têtes plus grosses aux deux extrémités de la prothèse. Cette liaison entre les fils n'empêche pas de réduire le diamètre de la prothèse par torsion en vue de sa mise en place, puisque le sens d'enroulement hélicoïdal est le même pour tous les fils. Le fait que les fils soient liés entre eux évite aussi le risque qu'un fil s'échappe accidentellement ou reste accidentellement dans le conduit corporel si l'on extrait la prothèse ultérieurement.

Un perfectionnement de la variante décrite ci-dessus consiste à réaliser plusieurs segments de fil, allant chacun d'une extrémité à l'autre de la prothèse, au moyen d'un même fil continu replié auxdites extrémités. La figure 9 représente un exemple d'une telle prothèse, réalisée au moyen d'un seul fil métallique continu, dont les deux extrémités ont été liées par une jonction 21 par exemple au moyen de soudure ou d'un manchon serti. Dans ce cas, le fil continu forme huit segments de fil 22, reliés deux à deux par quatre parties pliées 23 à chaque extrémité de la prothèse 20. Les segments de fil 22 sont enroulés en spires hélicoïdales qui sont toutes de même sens, ne se croisent jamais les unes les autres et ont un pas d'hélice plus court dans la partie médiane 6 que dans les autres parties 7 et 8 de la prothèse, comme on l'a décrit en référence à la figure 1. Les parties pliées assurent un bon accrochage de la prothèse 20 dans le conduit corporel où elle est placée, avec un moindre risque de traumatisme que dans le cas de la prothèse 5. De plus, comme chaque segment de fil 22 est lié aux autres, il ne risque pas de se déplacer indépendamment, ni de rester dans le conduit lorsqu'on enlève la prothèse.

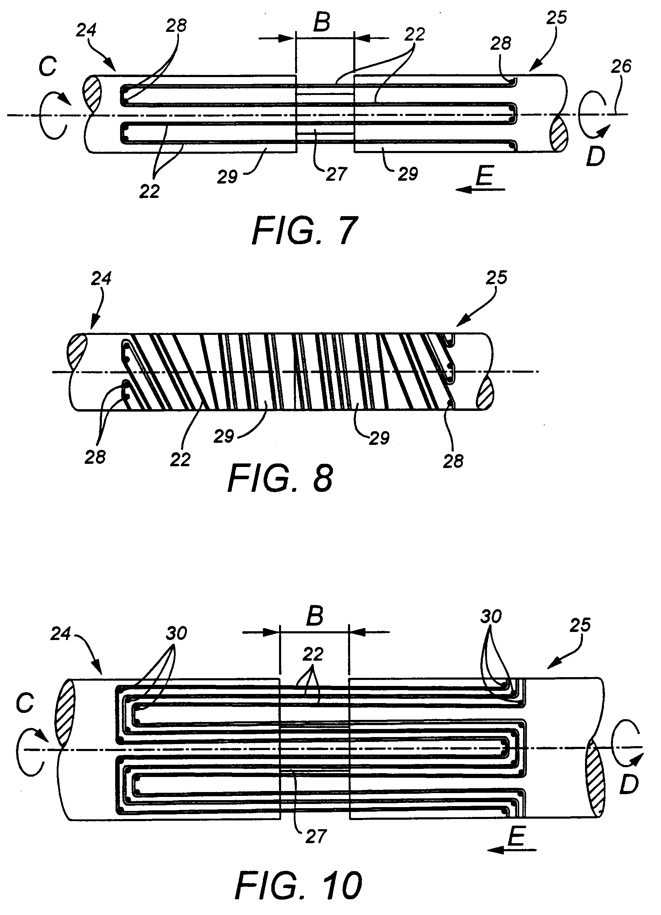

Les figures 7 et 8 illustrent un procédé et un dispositif pour la fabrication de la prothèse 20 de la figure 9. Le dispositif comporte un premier support cylindrique 24 et un second support cylindrique 25 qui sont alignés coaxialement sur un axe longitudinal 26 et sont reliés de manière coulissante au moyen d'un tenon cylindrique intérieur 27. Chaque support 24, 25 comporte une rangée circulaire de quatre paires d' ergots saillants 28, réparties sur le pourtour du support. Bien entendu les deux ergots 28 d'une paire peuvent être remplacés par un seul élément saillant remplissant les mêmes fonctions. Entre les deux séries d' ergots 28, le corps de chaque support 24, 25 constitue un mandrin cylindrique 29 sur lequel s'appuie le fil. Pour fabriquer la prothèse 20, on commence par placer les supports 24, 25 dans la position écartée de la figure 7, où un écart axial déterminé B est maintenu entre les extrémités des supports se faisant face. On dépose le fil métallique continu suivant un tracé en zig-zag entre les paires respectives d'ergots 28 des deux supports et en pliant le fil à angle droit sur chaque ergot 28 comme le montre la figure 7, pour former huit segments de fil 22 parallèles à l'axe 26. Une fois que le fil forme un parcours fermé on coupe ses extrémités et on les fixe ensemble. L'étape suivante consiste à tordre l'ensemble pour donner une forme hélicoïdale aux segments 22, par un mouvement de rotation relative des supports 24 et 25 suivant les flèches C et D combiné avec un rapprochement axial des supports suivant la flèche E. Comme les segments sont libres dans l'intervalle initial correspondant à B, ils s'enroulent plus rapidement dans cette zone à cause de la friction sur le mandrin 29, ce qui donne un pas d'hélice plus court dans la partie médiane 6 de la prothèse 20. A la fin de cette étape de torsion, dans la position illustrée par la figure 8, les deux supports 24 et 25 butent axialement l'un contre l'autre.

La forme hélicoïdale des segments de fil 22 se maintient grâce à l'écrouissage du fil lors de l'opération de torsion. On peut aussi envisager un traitement thermique dans ce but. Pour libérer la prothèse des ergots 28, il suffit d'une rotation relative des supports 24 et 25 dans le sens opposé.

La figure 10 illustre une variante du dispositif des figures 7 et 8, pour permettre de fabriquer la prothèse en déposant plusieurs fils métalliques en même temps, à savoir trois fils dans cet exemple. Chaque ergot 28 (figure 7) est alors remplacé par trois ergots 30 alignés dans une direction oblique et servant à plier à 90° chacun un fil. Les trois fils sont déposés parallèlement, c'est-à-dire que les segments de fil 22 ne se croisent jamais entre les deux rangées d' ergots 30. Si les groupes de trois ergots 30 sont inclinés alternativement dans un sens et dans l'autre comme le montre le dessin, les fils ne se croisent pas non plus dans les zones d'extrémité de la prothèse. Toutefois un croisement des fils dans ces zones ne constituerait pas forcément un inconvénient et pourrait être admis pour simplifier le dépôt des fils sur les supports 24 et 25. L'étape suivante de torsion de la prothèse suivant les flèches C, D et E, s'effectue comme dans l'exemple précédent.

Une variante du procédé décrit ci-dessus peut utiliser les mêmes dispositifs, mais dans la position rapprochée illustrée par la figure 8, pour déposer le fil métallique directement le long des trajectoires hélicoïdales voulues des segments de fil 22, grâce à des déplacements relatifs entre les supports 24 et 25 d'une part, et l'organe qui délivre le ou les fils d'autre part. Par exemple, ledit organe peut être fixe tandis que les supports 24 et 25 effectuent ensemble des mouvements hélicoïdaux en va-et-vient de sens opposés. A chaque extrémité de la prothèse, un petit mouvement de rotation supplémentaire fait passer le fil d'un ergot 28 ou 30 au suivant. On obtient ainsi la disposition représentée à la figure 8.

La présente invention n'est pas limitée aux exemples de réalisation et d'application décrits ci-dessus mais s'étend à toute modification ou variante évidente pour un homme du métier. Les fils peuvent avoir n'importe quelle section appropriée telle que ronde, ellipsoïdale, aplatie, etc. Les fils constituant la prothèse peuvent être faits de toute matière appropriée à l'utilisation prévue notamment des métaux tels que les alliages inoxydables biocompatibles, des alliages superélastiques ou à mémoire de forme, au titane, tantale, niobium, platine etc., mais aussi des matières synthétiques présentant les caractéristiques voulues. La structure à hélices multiples sans croisement des prothèses selon l'invention a l'avantage particulier de permettre l'emploi de fils métalliques pourvus d'un revêtement superficiel spécialement destiné à favoriser la tolérance de la prothèse dans le conduit concerné et/ou à empêcher la formation de dépôts, par exemple de plaquettes sanguines ou de graisse. Comme les segments de fil ne se croisent pas ce revêtement délicat n'est pas soumis à l'érosion par friction qu'il subit dans les "stents" classiques tissés ou tressés. Outre les vaisseaux sanguins les domaines d'application d'une telle prothèse comprennent divers conduits du corps humain ou animal tels que les conduits urinaires, génitaux, digestifs, pulmonaires, nasaux, auditifs ou autres.

## Revendications

1. Prothèse élastique pour élargir un conduit d'un être vivant, notamment un vaisseau sanguin la prothèse ayant une forme générale allongée sensiblement cylindrique et étant composée de plusieurs segments de fil élastique (1 2, 3, 4, 22) ayant une configuration en spires hélicoïdales, caractérisée en ce que le sens des spires est le même pour tous les segments de fil, et en ce qu'en dehors des zones d'extrémité de la prothèse, les segments de fil (1, 2, 3, 4, 22) sont intercalés les uns entre les autres sans se croiser, de manière à former ensemble ladite forme générale sensiblement cylindrique.

2. Prothèse selon la revendication 1 caractérisée en ce que les spires de chaque segment de fil ont un pas d' hélice (P₁) plus court dans une partie médiane (6) que dans le reste (7, 8) de la longueur de la prothèse.

3. Prothèse selon la revendication 2, caractérisée en ce que le pas d'hélice (P₂) dans le reste (7, 8) de la longueur vaut au moins 1,5 fois le pas d'hélice (P₁) dans la partie médiane (6).

4. Prothèse selon la revendication 1, caractérisée en ce que tous les segments de fils (1, 2, 3, 4, 22) sont identiques.

5. Prothèse selon la revendication 1, caractérisée en ce que les segments de fil (1, 2, 3, 4) ont chacun deux extrémités libres (10, 11) et ne sont pas liés les uns aux autres.

6. Prothèse selon la revendication 5, caractérisée en ce qu'à une extrémité de la prothèse (5), les extrémités libres (10, 11) des segments de fil se trouvent sensiblement dans un même plan transversal.

7. Prothèse selon la revendication 5, caractérisée en ce qu'à une extrémité de la prothèse (5'), les extrémités libres (11) des segments de fil sont décalées axialement les unes par rapport aux autres.

8. Prothèse selon l'une des revendications précédentes, caractérisée en ce que le nombre de segments de fil (1, 2, 3, 4, 22) est compris entre 4 et 18.

9. Prothèse selon la revendication 1, caractérisée en ce que les segments de fil (22) sont liés mutuellement au moins deux à deux aux extrémités de la prothèse (20).

10. Prothèse selon la revendication 9, caractérisée en ce que lesdits segments de fil (22) sont liés par soudage.

11. Prothèse selon la revendication 9, caractérisée en ce qu'au moins certains des segments de fil (22) sont formés d'un seul fil continu qui est replié ou recourbé à 180° à chaque extrémité de la prothèse pour raccorder à chaque fois deux desdits segments.

12. Procédé de fabrication d'une prothèse selon la revendication 11 à partir d'un fil métallique continu, caractérisé par les étapes suivantes :
- on utilise des moyens de support comprenant un premier et un second support (24, 25) à pourtour circulaire, qui sont alignés axialement l'un en face de l'autre et ont chacun une série d'éléments saillants (28, 30) répartis sur le pourtour du support, et un mandrin central (29) disposé axialement entre les deux séries d'éléments saillants;
- on dépose le fil sur les moyens de support suivant un tracé en zig-zag par un mouvement en va-et-vient axial entre les deux série d'éléments saillants (28, 30) pour former un ensemble de segments de fil axiaux (22), parallèles et répartis autour du mandrin, le fil étant replié successivement sur au moins un élément saillant du premier support, puis du second support, puis sur au moins un autre élément saillant du premier support, et ainsi de suite;
- on coupe le fil pour former deux extrémités adjacentes et on fixe lesdites extrémités l'une à l'autre;
- on tord ledit ensemble de segments de fil (22) autour du mandrin (29) pour donner à chaque segment de fil une configuration permanente en spires hélicoïdales et l'on enlève des moyens de support ledit ensemble qui forme la prothèse (20).

13. Procédé de fabrication d'une prothèse selon la revendication 11 à partir d'un fil métallique continu, caractérisé par les étapes suivantes :
- on utilise des moyens de support comprenant un premier et un second support (24, 25) à pourtour circulaire, qui sont alignés axialement l'un en face de l'autre et ont chacun une série d'éléments saillants (28, 30) répartis sur le pourtour du support, et un mandrin central (29) disposé axialement entre les deux séries d'éléments saillants;
- on dépose le fil sur les moyens de support suivant un tracé en zig-zag par un mouvement en va-et-vient hélicoïdal entre les deux séries d'éléments saillants (28, 30), ou par un mouvement en va-et-vient axial combiné avec un mouvement alternatif de rotation des moyens de support, pour former un ensemble de segments de fil parallèles et enroulés en spires hélicoïdales autour du mandrin, le fil étant replié successivement sur au moins un élément saillant du premier support, puis du second support, puis sur au moins un autre élément saillant du premier support et ainsi de suite;
- on coupe le fil pour former deux extrémités adjacentes et on fixe lesdites extrémités l'une à l'autre; et
- on enlève des moyens de support ledit ensemble qui forme la prothèse (20).

## Claims

1. Elastic prosthesis for widening a conduit in a living being, in particular a blood vessel, the prosthesis being of a substantially elongated and cylindrical shape and comprising several elastic wire segments (1, 2, 3, 4, 22) having a helical spiral configuration, characterised in that the direction of the spirals is the same for all the wire segments, and in that outside the regions of extremity of the prosthesis, the wire segments (1, 2, 3, 4, 22) are intercalated with one another without crossing so as to form the said substantially cylindrical shape.

2. Prosthesis according to claim 1, characterised in that the spirals of each wire segment have a shorter helical pitch (P₁) in the median section (6) than in the remaining (7, 8) length of the prosthesis.

3. Prosthesis according to claim 2, characterised in that the helical pitch (P₂) in the remaining length (7, 8) is at least 1.5 times the helical pitch (P₁) in the median section (6).

4. Prosthesis according to claim 1, characterised in that all the wire segments (1, 2, 3, 4, 22) are identical.

5. Prosthesis according to claim 1, characterised in that the wire segments (1, 2, 3, 4) each have two free extremities (10, 11) and are not connected to one another.

6. Prosthesis according to claim 5, characterised in that at an extremity of the prosthesis (5), the free extremities (10, 11) of the wire segments are substantially in the same transversal plane.

7. Prosthesis according to claim 5, characterised in that at an extremity of the prosthesis (5'), the free extremities (11) of the wire segments are staggered axially in relation to one another.

8. Prosthesis according to any one of the preceding claims, characterised in that the number of wire segments (1, 2, 3, 4, 22) is between 4 and 18 inclusive.

9. Prosthesis according to claim 1, characterised in that the wire segments (22) are mutually connected mutually at least two at a time at the extremities of the prosthesis (20).

10. Prosthesis according to claim 9, characterised in that the said wire segments (22) are connected by welding.

11. Prosthesis according to claim 9, characterised in that at least some of the wire segments (22) are formed from a single continuous wire which is folded or bent through 180° at each extremity of the prosthesis in order to connect in each case two of the said segments.

12. Method of manufacturing a prosthesis according to claim 11 from a continuous metal wire, characterised by the following stages:
- means of support comprising a first and a second support (24, 25) with a circular periphery and aligned axially one facing the other and each having a series of protruding elements (28, 30) distributed along the periphery of the support, and a central mandrel (29) arranged axially between the two series of protruding elements are used;
- the wire is placed on the means of support zigzag fashion using an axial zigzag movement between the two series of protruding elements (28, 30) in order to form an assembly of axial wire segments (22) which are parallel and distributed around the mandrel, the wire being folded successively around at least one protruding element of the initial support and then of the second support, then around at least one other protruding element of the initial support and so on;
- the wire is cut to form two adjacent extremities and the said extremities are fixed one to the other;
- the said assembly of wire segments (22) is then twisted around the mandrel (29) to confer upon each wire segment a permanent helical spiral configuration and the said assembly which forms the prosthesis (20) is removed from the means of support.

13. Method of manufacturing a prosthesis according to claim 11 from a continuous metal wire, characterised by the following stages:
- support means comprising a first and a second support (24, 25) with a circular periphery and aligned axially one facing the other and each having a series of protruding elements (28, 30) distributed around the periphery of the support, and a central mandrel (29) arranged axially between the two series of protruding elements are used;
- the wire is placed on the means of support zigzag fashion using a helical zigzag movement between the two series of protruding elements (28, 30), or by an axial zigzag movement combined with an alternating movement of rotation of the means of support in order to form a parallel assembly of wire segments wound round in helical spirals around the mandrel, the wire being folded successively round at least one protruding element of the initial support, then of the second support, then round at least one protruding element of the initial support and so on;
- the wire is cut to form two adjacent extremities and the said extremities are fixed one to the other; and
- the said assembly, which forms the prosthesis (20), is removed from the means of support.

## Patentansprüche

1. Elastische Prothese zur Erweiterung eines Gefäßes eines lebendigen Wesens, insbesondere eines Blutgefäßes, wobei die Prothese eine verlängerte, wenigstens annähernd zylindrische Form aufweist und aus mehreren Segmenten elastischer Fäden (1,2,3,4, 22) besteht, die eine Konfiguration in Form von schraubenförmigen Windungen aufweist,
**dadurch gekennzeichnet,** daß
die Richtung der Windungen bei allen Fadensegmenten gleich ist, und dadurch, daß die Fadensegmente (1,2,3,4,22) außerhalb der Endzonen der Prothese jeweils ohne sich zu kreuzen so ineinander eingeschoben sind, daß sie gemeinsam die wenigstens annähernd, zylindrisch ausgeprägte Form bilden.

2. Prothese nach Anspruch 1,
**dadurch gekennzeichnet,** daß
die Windungen eines jeden Fadensegmentes eine kürzere Steigung (P₁) in einem Mittelteil (6) aufweisen, als im Rest (7,8) der Länge der Prothese.

3. Prothese nach Anspruch 2,
**dadurch gekennzeichnet,** daß
die Steigung (P₂) im Rest (7,8) der Länge mindestens das 1,5-fache der Steigung (P₁) im Mittelteil (6) beträgt.

4. Prothese nach Anspruch 1,
**dadurch gekennzeichnet,** daß
alle Fadensegmente (1,2,3,4,22) identisch sind.

5. Prothese nach Anspruch 1,
**dadurch gekennzeichnet,** daß
die Fadensegmente (1,2,3,4) jeweils zwei freie Enden (10,11) aufweisen und nicht miteinander verbunden sind.

6. Prothese nach Anspruch 5,
**dadurch gekennzeichnet,** daß
sich die freien Enden (10,11) der Fadensegmente an einem Ende der Prothese (5) wenigstens annähernd in einer gleichen transversalen Ebene befinden.

7. Prothese nach Anspruch 5,
**dadurch gekennzeichnet,** daß
die freien Enden (11) der Fadensegmente an einem Ende der Prothese (5) im Verhältnis zueinander axial versetzt sind.

8. Prothese nach einem der vorhergehenden Ansprüche,
**dadurch gekennzeichnet,** daß
die Anzahl der Fadensegmente (1,2,3,4,22) zwischen 4 und 18 liegt.

9. Prothese nach Anspruch 1,
**dadurch gekennzeichnet,** daß
die Fadensegmente (22) gegenseitig mindestens paarweise an den Enden der Prothese (20) miteinander verbunden sind.

10. Prothese nach Anspruch 9,
**dadurch gekennzeichnet,** daß
die Fadensegmente (22) durch Schweißen miteinander verbunden sind.

11. Prothese nach Anspruch 9,
**dadurch gekennzeichnet,** daß
mindestens einige der Fadensegmente (22) aus einem zusammenhängenden Faden gebildet sind, der an jedem Ende der Prothese umgeschlagen oder um 180° umgebogen ist, um jedes Mal zwei der Fadensegmente zu verbinden.

12. Verfahren zur Herstellung einer Prothese nach Anspruch 11 aus einem fortlaufenden metallischen Faden,
**gekennzeichnet durch**
die folgenden Verfahrensschritte:
- es werden Trägermittel verwendet, die einen ersten und einen zweiten Träger (24,25) mit kreisförmigem Umfang aufweisen, wobei diese axial einander gegenüberliegend ausgerichtet sind, und jeweils eine Reihe von hervorstehenden Elementen (28,30) aufweisen, die über den Umfang des Trägers verteilt sind, sowie einen Mittelspanndorn (29) aufweist, der zwischen den beiden Reihen von hervorstehenden Elementen axial angeordnet ist;
- der Faden auf den Trägermitteln in Form eines Zick-Zack-Musters durch eine axiale Hin- und Herbewegung zwischen den beiden Reihen hervorstehender Elemente (28,30) aufgelegt wird, um eine Einheit aus axialen Fadensegmenten (22) zu bilden, die parallel und rund um den Spanndorn angeordnet sind, wobei der Faden aufeinanderfolgend um mindestens ein hervorstehendes Element des ersten Trägers umgeschlagen wird, und nachfolgend;
- der Faden geschnitten wird, um zwei aneinander angrenzende äußerste Enden zu bilden, und die äußersten Enden aneinander befestigt werden;
- die Einheit von Fadensegmenten (22) um den Spanndorn (29) herum verdrillt wird, um jedem Fadensegment eine dauerhafte Form in Gestalt von schraubenförmigen Windungen zu verleihen, und die Trägermittel von der Einheit entfernt werden, die die Prothese (20) bildet.

13. Verfahren zur Herstellung einer Prothese nach Anspruch 11 aus einem fortlaufenden metallischen Faden,
**gekennzeichnet durch**
die folgenden Etappen:
- es werden Trägermittel verwendet, die einen ersten und einen zweiten Träger (24,25) mit kreisförmigem Umfang aufweisen, wobei diese axial einander gegenüberliegend ausgerichtet sind, und jeweils eine Reihe von hervorstehenden Elementen (28,30) aufweisen, die über den Umfang des Trägers verteilt sind, sowie einen Mittelspanndorn (29) aufweist, der zwischen den beiden Reihen von hervorstehenden Elementen axial angeordnet ist;
- der Faden auf den Trägermitteln in Form eines Zick-Zack-Musters durch eine axiale Hin- und Herbewegung zwischen den beiden Reihen hervorstehender Elemente (28,30), oder durch eine axiale Hin- und Herbewegung in Kombination mit einer wechselweisen Drehbewegung, aufgelegt wird, um eine Einheit aus Fadensegmenten (22) zu bilden, die parallel und in schraubenförmigen Windungen rund um den Spanndorn angeordnet sind, wobei der Faden aufeinanderfolgend um mindestens ein hervorstehendes Element des ersten Trägers, und dann des zweiten Trägers umgeschlagen wird, und nachfolgend;
- der Faden geschnitten wird, um zwei aneinander angrenzende äußerste Enden zu bilden, und die äußersten Enden aneinander befestigt werden; und
- die Einheit, die die Prothese (20) bildet, von den Trägermitteln entfernt wird.
